# FASCICULE DE BREVET EUROPEEN

(11) **EP 2 417 186 B1**
(45) Date de publication et mention de la délivrance du brevet: **02.07.2014**
(21) Numéro de dépôt: 10710058.8
(22) Date de dépôt: 25.03.2010
(51) Int. Cl.: C08G 69/28, C07C 51/41

(54) **PROCEDE DE FABRICATION D'UNE SOLUTION DE SELS DE DIACIDES/DIAMINE(S)**
VERFAHREN ZUR HERSTELLUNG EINER LÖSUNG VON DISÄURE/DIAMIN-SALZEN
METHOD FOR MANUFACTURING A SOLUTION OF DIACID/DIAMINE SALTS

(30) Priorité: 09.04.2009 FR 0952333
(43) Date de publication de la demande: 15.02.2012
(73) Titulaire: Rhodia Opérations, 93306 Aubervilliers (FR)
(72) Inventeur: LOMEL, Sébastien, F-38540 Saint Just Chaleyssin (FR); THIERRY, Jean-François, F-69340 Francheville (FR); BOSSENNEC, Véronique, F-69360 Serezin-du-Rhône (FR)
(86) Numéro de dépôt international: PCT/EP2010/053946
(87) Numéro de publication internationale: WO 2010/115727

(56) Documents cités:
- EP-A2- 0 812 869
- GB-A- 830 676
- JP-A- 2008 239 908
- US-A- 4 118 351
- US-A- 5 028 462
- US-A- 5 891 987
- US-B1- 6 284 830

## Description

La présente invention concerne un procédé de fabrication d'une solution d'un sel de diamine(s) et de diacides pour la fabrication de polyamide.

Pour obtenir des polyamides à monomères diacides et diamines de poids moléculaire élevé, on utilise généralement une solution aqueuse d'un sel formé par réaction entre une molécule de diamine et une molécule de diacide. Cette solution est chauffée pour évaporer l'eau dans un premier temps puis pour engager la polymérisation par polycondensation, pour obtenir des chaînes macromoléculaires comprenant des fonctions amides.

La solution de sel contient généralement des quantités essentiellement stoechiométriques de diacide(s) et diamine(s). La concentration pondérale en sel Nylon (sel adipate d'hexaméthylène diammonium), utilisé comme matière première pour la fabrication de polyamide, plus précisément de PA66, est généralement comprise entre 50% et 65%. Cette solution est généralement stockée avant d'être transportée le cas échéant, puis alimentée dans les installations de polymérisation.

Plusieurs procédés de fabrication de solution de sel de diacide(s) et de diamine(s) ont été proposés. Pour la fabrication de sel Nylon, ces procédés consistent généralement à ajouter l'acide adipique dans l'hexaméthylène diamine, ou l'inverse, en milieu aqueux, en évacuant ou non la chaleur produite par la réaction de neutralisation.

Pour la fabrication de copolyamides, en particulier de copolyamides à partir de diacides aromatiques, d'acide adipique et d'hexaméthylène diamine, un certain nombre de monomères de nature différente sont mis en oeuvre. En amont de la réaction de polymérisation de ces copolyamides, lors de la préparation de la solution de sel de diacides et de diamine(s) à partir de ces différents monomères, on rencontre des problèmes de dissolution de ces différents monomères. Par exemple la dissolution est parfois impossible, ou le temps de dissolution est très long, ou l'on doit mettre en oeuvre des solutions très diluées. Ceci peut engendrer des problèmes de productivité, de qualité du produit, de stockage et transport de la solution dans l'installation de polymérisation.

On recherche donc des procédés de préparation de solution d'acides et de diamine(s) ne présentant pas ces problèmes.

A cet effet, l'invention propose un procédé de fabrication d'une solution aqueuse (A) de sels de diacides et diamine(s) obtenus par mélange d'au moins deux diacides et d'au moins une diamine à une concentration pondérale en sel comprise entre 40 et 70% caractérisé en ce qu'il comprend les étapes suivantes :
- produire dans un réacteur une solution aqueuse (A') de diamine(s) et diacide(s), ayant un rapport molaire diacide/diamine inférieur à 1, de préférence inférieur ou égal à 0,9, par alimentation dans ledit réacteur contenant un liquide à une température comprise entre 55 et 95°C (bornes incluses), de préférence entre 60 et 90°C (bornes incluses) comprenant de l'eau et de la diamine, d'un flux (B') comprenant un diacide, éventuellement d'un flux comprenant de la diamine, éventuellement d'un flux comprenant de l'eau ; les débits du ou des flux d'alimentation étant contrôlé(s) pour avoir constamment une température de la solution dans le réacteur inférieure à la température d'ébullition sous la pression opératoire de celle-ci ; les quantités d'eau et de diamine du liquide ainsi que les débits des flux d'alimentation étant contrôlés pour avoir constamment un rapport molaire diacide(s)/diamine(s) inférieur à 1 ; le diacide du flux (B') étant un diacide aliphatique ou cycloaliphatique comprenant un nombre d'atomes de carbone supérieur à 10 ou un diacide aromatique ;
- mélanger la solution aqueuse (A') issue de la première étape avec un flux (B") comprenant au moins un diacide, le diacide étant un diacide aliphatique ou cycloaliphatique comprenant un nombre d'atomes de carbone inférieur ou égal à 10, et éventuellement de l'eau et/ou de la diamine complémentaires ; afin d'obtenir une solution aqueuse (issue du mélange de (A') et (B")) ayant un rapport molaire diacides/diamine(s) compris entre 0,9 et 1,1 , de préférence compris entre 0,98 et 1,02 (bornes comprises) ; cette solution étant portée à une température au plus égale à la température d'ébullition de la solution à la pression opératoire par au moins le dégagement de chaleur de la réaction entre la(les) diamine(s) et les diacides ; ceci afin d'obtenir la solution (A) de diacides et diamine(s) à la concentration et à la composition désirées.

Par température d'ébullition, il faut comprendre la température d'ébullition de la solution contenue dans un réacteur à la pression de travail ou opératoire du procédé.

Comme diamines convenables pour l'invention on peut citer l'hexaméthylène diamine (HMD) comme monomère préféré et le plus utilisé, et également l'heptaméthylène diamine, la tétraméthylène diamine, l'octaméthylène diamine, la nonaméthylène diamine, la décaméthylène diamine, la méthyl-2 pentaméthylène diamine, l'undécaméthylène diamine, la dodécaméthylène diamine, la xylylène diamine, l'isophorone diamine. Il est possible d'utiliser un mélange de plusieurs monomères diamines.

Dans le procédé de l'invention, la diamine peut être mise en oeuvre sous forme pure ou sous forme de solution aqueuse concentrée. Pour l'HMD, une solution comprenant au moins 50% en poids de diamine, de préférence au moins 85% et encore plus avantageusement 90% en poids environ peut être utilisée. Toutefois, les flux comprenant la diamine peuvent contenir d'autres composés sans pour cela sortir du cadre de l'invention.

Comme diacide aromatique, ou diacide aliphatique ou cycloaliphatique comprenant un nombre d'atomes de carbone supérieur à 10, convenable pour l'invention, on peut citer l'acide dodécanedioïque, isophtalique, téréphtalique, naphtalène dicarboxylique par exemple. Le diacide préféré est l'acide téréphtalique.

Comme diacide aliphatique ou cycloaliphatique comprenant un nombre d'atomes de carbone inférieur ou égal à 10, convenable pour l'invention, on peut citer l'acide adipique, subérique, sébacique, azélaïque, pimélique, l'acide cyclohexane dicarboxylique. Le diacide préféré est l'acide adipique.

Les diacides sont généralement utilisés sous forme de poudre. Toutefois, il peuvent également être sous forme de solution aqueuse ou de suspension.

Avantageusement on met en oeuvre un mélange de diacide aromatique et de diacide aliphatique. La proportion molaire de diacide aromatique par rapport au diacide aliphatique est comprise entre 5 et 80%, de préférence entre 20 et 50%. De préférence le diacide aromatique est l'acide téréphtalique, et le diacide aliphatique est l'acide adipique. Avantageusement la proportion molaire d'acide téréphtalique par rapport aux diacides est comprise entre 5 et 80%, de préférence entre 20 et 50%.

Comme pour les flux comprenant la diamine, les flux comprenant le diacide peuvent contenir d'autres composés, tels que des solvants, sans pour autant sortir du cadre de l'invention.

Des limiteurs de chaîne peuvent être introduits durant la mise en oeuvre du procédé de l'invention, par exemple lors de la première étape du procédé de préparation de la solution aqueuse (A') ou dans le flux (B"). A titre d'exemple de limiteurs de chaînes on peut citer par exemple l'acide benzoïque, l'acide acétique etc.

La première étape du procédé de l'invention consiste à préparer une solution aqueuse (A') de diamine(s) et diacide(s), ayant un rapport molaire diacide/diamine inférieur à 1, de préférence inférieur ou égal à 0,9.

La préparation de cette solution aqueuse (A') consiste à alimenter dans un liquide comprenant de l'eau et de la diamine, un flux (B') comprenant le diacide. Avantageusement l'eau et la diamine du liquide représentent la totalité de l'eau et de la diamine de la solution (A'). Lorsque l'eau et la diamine du liquide ne représentent pas la totalité de la diamine et de l'eau du liquide, un flux de diamine et/ou un flux d'eau peuvent également être alimentés dans le réacteur, en plus du flux (B') comprenant le diacide. La diamine du liquide (A') est avantageusement l'hexaméthylène diamine.

La concentration en eau dans le liquide peut varier dans un large domaine. Toutefois cette dernière est suffisamment élevée pour permettre la dissolution du diacide du flux (B') et donc l'obtention de la solution (A') dans un délai raisonnable. Avantageusement la concentration en eau du liquide est supérieure ou égale à 30% en poids.

Le liquide est à une température comprise entre 55 et 95°C (bornes comprises), de préférence entre 60 et 90°C (bornes comprises). Ceci permet de se placer dans les meilleures conditions pour procéder à la dissolution du diacide du flux (B'). Le liquide peut être chauffé ou non.

Le liquide peut aussi comprendre une partie du diacide aliphatique ou cycloaliphatique ayant un nombre d'atomes de carbone inférieur ou égal à 10, correspondant ou non au diacide introduit dans le flux (B").

Selon un mode de réalisation particulier du procédé de l'invention et lorsque le procédé est discontinu, le réacteur, en début de première étape du procédé, peut contenir une faible quantité de solution aqueuse (A) ou de solution aqueuse (A'), appelée pied de solution. Cette solution aqueuse est une faible partie de la solution (A) ou de la solution (A') préparée dans une opération antérieure.

La quantité d'une telle solution (A) ou solution (A') présente dans le réacteur en début de première étape est égale à au moins environ 5%, avantageusement entre 5 et 40%, de préférence entre 10% et 35% en poids de la quantité totale de solution (A) ou de la solution (A') à produire dans le réacteur.

Avantageusement, selon une caractéristique de l'invention on minimise les échanges de chaleur entre le réacteur et l'environnement, ou l'extérieur, c'est-à-dire que le réacteur fonctionne en mode quasi adiabatique.

La température dans le réacteur lors de l'alimentation du flux (B') peut s'élever à cause de la réaction de neutralisation entre la diamine et le diacide. Toutefois, la température de la solution dans le réacteur, pendant toute l'opération et en fin d'étape, sera toujours inférieure à la température d'ébullition de la solution sous la pression opératoire.

Le diacide du flux (B') est un diacide aromatique, ou un diacide aliphatique ou cycloaliphatique comprenant plus de 10 atomes de carbone. Il s'agit avantageusement de l'acide téréphtalique.

La concentration en eau de la solution (A') peut varier dans un large domaine, en fonction de la concentration du sel final souhaitée, et donc de la quantité d'eau éventuellement apportée par le flux (B").

Selon un mode de réalisation particulier du procédé de l'invention, la température de la solution (A') est supérieure ou égale à 75°C, afin d'éviter toute apparition de phase solide dans la solution (A').

La mise en oeuvre de la première étape du procédé de l'invention permet de réaliser une très bonne dissolution du diacide du flux (B').

Dans une deuxième étape du procédé de l'invention, on mélange la solution (A') issue de la première étape avec un flux (B") comprenant au moins du diacide, et éventuellement de l'eau et/ou de la diamine complémentaires. Ceci permet d'obtenir une solution aqueuse (issue du mélange de (A') et (B")) ayant un rapport molaire diacides/diamine(s) compris entre 0,9 et 1,1, de préférence compris entre 0,98 et 1,02 (bornes comprises). Cette étape permet l'obtention d'une solution de diacides et diamines à la concentration et à la composition désirées.

De l'eau peut être également ajoutée pour ajuster la concentration en sel de diacide(s)/diamine(s) à une concentration pondérale supérieure à 40%, de préférence comprise entre 50 et 65% en poids. L'eau peut être avantageusement mélangée au flux d'acide.

Le diacide du flux (B") est un diacide aliphatique ou cycloaliphatique comprenant un nombre d'atomes de carbone inférieur ou égal à 10. Il s'agit avantageusement de l'acide adipique.

Avantageusement le flux (B") est une solution aqueuse de diacide et de diamine avec un rapport molaire diacide/diamine compris entre 1,5 et 5 et une concentration dans l'eau des espèces dissoutes comprise entre 40 et 75%, de préférence entre 45 et 65%. La diamine du flux (B") est avantageusement l'hexaméthylène diamine. La température du flux (B") est suffisamment élevée pour éviter toute apparition de phase solide dans le flux (B"). La chaleur de la réaction de neutralisation de l'amine par l'acide provoque une augmentation de la température dans le réacteur de préparation du flux (B"), pour atteindre au maximum la température d'ébullition du mélange à la pression opératoire.

Par espèces dissoutes, il faut comprendre l'ensemble des espèces diacides et diamines présentes dans le milieu sous forme libre ou ionique (sel) ou autre.

Selon un mode de réalisation particulier du procédé de l'invention, et lorsque le procédé est discontinu, le réacteur de préparation du flux (B"), en début de préparation du flux (B"), peut contenir une faible quantité de flux (B"), appelée pied. Ce pied est une faible partie du flux (B") préparé dans une opération antérieure.

La quantité d'un tel pied présent dans le réacteur en début de préparation du flux (B") est égale à au moins environ 5%, avantageusement entre 5 et 40%, de préférence entre 10% et 35% en poids de la quantité totale de flux (B") à produire dans le réacteur.

Avantageusement, selon une caractéristique de l'invention on minimise les échanges de chaleur entre le réacteur et l'environnement, ou l'extérieur, c'est-à-dire que le réacteur fonctionne en mode quasi adiabatique.

Selon un mode de réalisation particulier du procédé de l'invention, le procédé de l'invention comprend une étape supplémentaire d'ajustement du rapport molaire diacides/diamine, par exemple à une valeur comprise entre 0,995 et 1,005. Cette étape est de préférence réalisée par ajout de diamine et d'eau.

Le procédé de l'invention est réalisé avantageusement en maintenant le(s) réacteur(s) sous atmosphère exempte d'oxygène, comme par exemple, sous une atmosphère constituée par de l'azote, des gaz rares, de la vapeur d'eau ou un mélange de ceux-ci.

Dans un mode de réalisation préféré, l'atmosphère exempte d'oxygène est obtenue soit par alimentation en continu d'un flux d'azote, soit par maintien d'une pression d'azote dans le(s) réacteur(s) et par génération de vapeur d'eau par l'ébullition de la solution.

Dans ce dernier cas, il est avantageux que l'échappement ou évacuation de l'azote, soit réalisé à travers un condenseur monté sur le(s) réacteur(s). Ainsi, l'eau entraînée avec l'azote est condensée et recyclée dans le(s) réacteur(s).

Ce mode de réalisation permet également l'évacuation de l'oxygène présent, par exemple sous forme dissoute, dans la solution et donc évite une oxydation des monomères, notamment de la diamine. L'oxygène peut être apporté notamment par les monomères diacides.

Selon un autre mode de réalisation, le réacteur est maintenu sous atmosphère exempte d'oxygène par alimentation, par exemple, d'azote dans le réacteur vide, et maintien de cette atmosphère d'azote lors des remplissages et vidanges du réacteur.

Dans ce mode de réalisation, l'oxygène dissous sera évacué par entraînement par l'azote qui s'échappe du réacteur au cours du remplissage de celui-ci. Cette évacuation de l'azote est effectuée, de préférence, à travers un condenseur pour ainsi condenser la vapeur d'eau entraînée par l'azote.

De préférence le(s) réacteur(s) est(sont) muni(s) d'une isolation thermique pour limiter les échanges de chaleur avec le milieu extérieur et ainsi limiter les pertes de chaleur.

Le procédé de l'invention peut être réalisé selon un mode discontinu ou un mode continu. Ces deux modes de réalisation sont décrits en détail ci-dessous.

Le procédé de l'invention peut être mis en oeuvre dans tout type de réacteur. Plus particulièrement, les réacteurs dans lesquels sont introduits des matériaux solides comprennent une agitation mécanique et peuvent être équipés de moyens permettant de les maintenir en température, notamment pendant les périodes d'arrêt ou de changement de campagne de fabrication. Pour obtenir une homogénéisation de la solution dans le réacteur, une boucle de circulation externe comprenant une pompe peut être utilisée.

Le procédé de l'invention peut être mis en oeuvre dans un réacteur unique, ou dans plusieurs réacteurs. Ces réacteurs peuvent par exemple être montés en parallèle ou en série.

Selon le procédé de l'invention, le rapport molaire diacides/diamine peut être avantageusement contrôlé et ajusté par la mesure du pH de la solution et l'ajout de diacide et/ou diamine complémentaire en fonction du résultat de cette mesure de pH.

La solution de sel obtenue selon le procédé de l'invention peut être alimentée directement dans une installation de polymérisation, ou peut être stockée dans un réservoir de stockage ou dans des conteneurs adaptés pour le transport, avant éventuellement transfert, et utilisation.

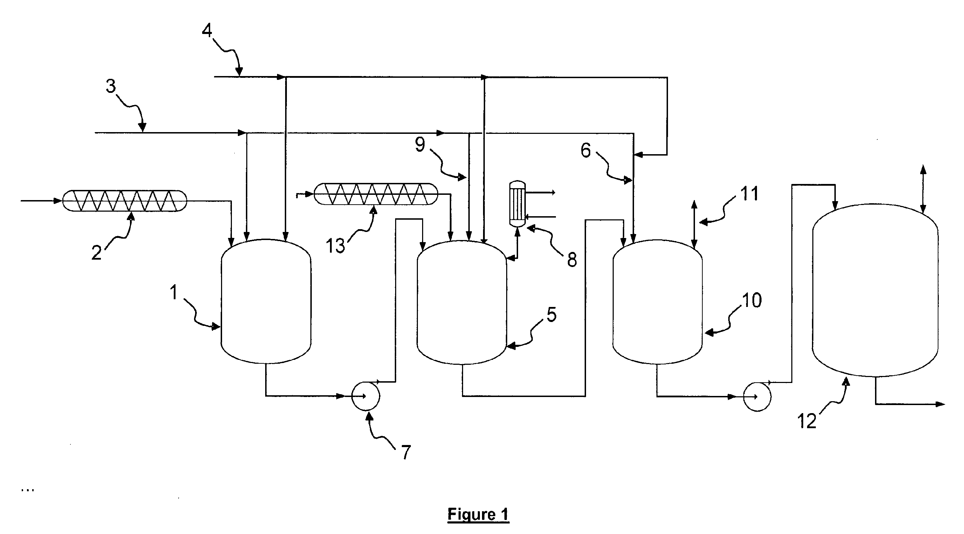

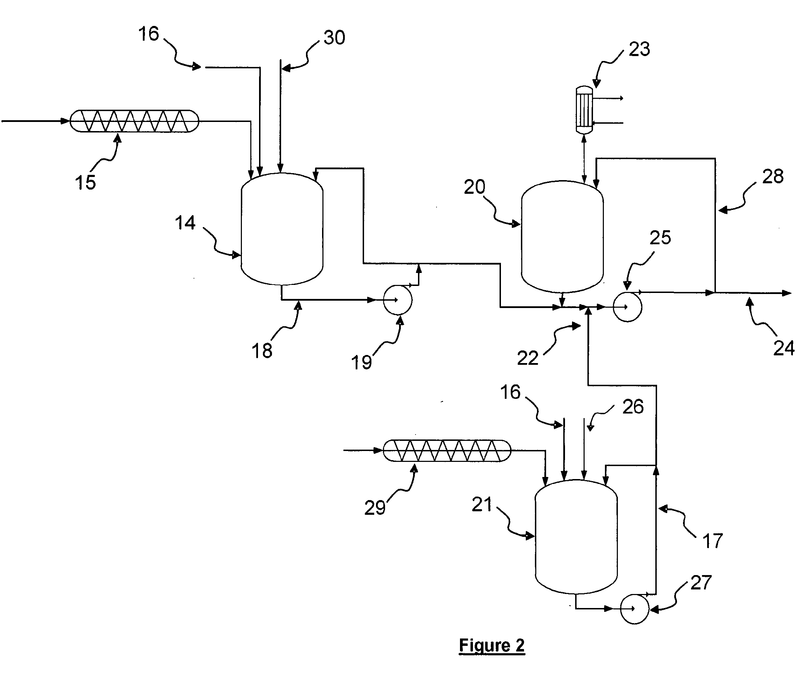

Une description détaillée de deux modes de réalisation du procédé de l'invention est réalisée ci-dessous en référence aux figures 1 et 2 annexées dans lesquelles :
- la figure 1 représente un schéma synoptique d'une installation permettant de mettre en oeuvre le procédé selon un mode de réalisation discontinu, et
- la figure 2 représente un schéma synoptique d'une installation permettant de mettre en oeuvre le procédé selon un mode de réalisation continu.

L'invention est également illustrée par les exemples de fabrication de solutions de sel obtenues selon le mode de réalisation en discontinu du procédé.

Dans la description ci-dessous on utilisera les termes acide adipique (AA), acide téréphtalique (AT) et hexaméthylène diamine (HMD) pour désigner les diacides et la diamine. Toutefois, ce procédé s'applique également à d'autres diacides et d'autres diamines indiqués précédemment.

En référence à la figure 1, on décrit un premier mode de réalisation du procédé de l'invention fonctionnant selon le mode discontinu. L'installation comprend un premier réacteur 1 agité, dans lequel sont ajoutés de l'acide adipique 2, généralement sous forme d'une poudre, et un flux 3 liquide d'hexaméthylène diamine. De l'eau 4 est également introduite dans ce réacteur.

Les différents produits sont ajoutés dans le réacteur 1 qui contient une faible quantité de solution d'acide adipique et d'hexaméthylène diamine dans de l'eau, riche en acide adipique, et appelée pied de solution. Cette solution aqueuse est avantageusement une faible partie de la solution préparée dans une opération antérieure et a, avantageusement, comme composition, sensiblement la composition finale de la solution qui sera préparée dans ce réacteur 1, à savoir un rapport molaire diacide/diamine égal à environ 2,4 et une concentration pondérale en espèces dissoutes d'environ 57%.

L'installation comprend également un deuxième réacteur agité 5, dans lequel sont ajoutés de l'eau 4, un flux 9 liquide d'hexaméthylène diamine, et de l'acide téréphtalique 13, généralement sous forme de poudre.

L'hexaméthylène diamine et l'eau sont ajoutés dans le réacteur 5 qui contient une faible quantité de solution d'acide téréphtalique, d'acide adipique et d'hexaméthylène diamine dans de l'eau, appelée pied de solution. Cette solution aqueuse est avantageusement une faible partie de la solution (A) préparée dans une opération antérieure et a, avantageusement, comme composition, sensiblement la composition finale de la solution (A) qui sera préparée dans ce réacteur 5, à savoir un rapport molaire diacide/diamine égal à environ 1,017 et une concentration pondérale en espèces dissoutes d'environ 52%. L'acide téréphtalique est ensuite introduit dans le réacteur 5. On obtient la dissolution complète de l'acide téréphtalique.

La solution du réacteur 1 est ensuite alimentée dans le second réacteur 5 par une pompe 7. Ce réacteur 5 est équipé d'un condenseur 8 et avantageusement d'une boucle externe de circulation de la solution et/ou d'un agitateur (non représentés).

Dans ce deuxième réacteur 5, la solution du réacteur 1 est alimentée pour obtenir un rapport molaire diacides/HMD voisin de 1,017. Comme pour le premier réacteur 1, aucun échange de chaleur significatif n'est avantageusement réalisé avec l'extérieur. Ainsi la chaleur de la réaction de neutralisation de l'amine par l'acide provoque une augmentation de la température dans le réacteur 5 pour atteindre au maximum la température d'ébullition du mélange à la pression opératoire. L'eau qui s'évapore est condensée dans le condenseur 8 pour obtenir un reflux total de l'eau.

Dans le mode illustré qui est le mode préféré de l'invention, la solution obtenue dans le deuxième réacteur 5 est alimentée dans un troisième réacteur 10 muni d'un moyen d'homogénéisation (non représenté) et éventuellement d'un condenseur 11.

Ce troisième réacteur 10, appelé également réacteur d'ajustement, comprend une addition 6 d'HMD et d'eau pour ajuster le rapport diacides/HMD à une valeur par exemple comprise entre 0,995 et 1,005, et ajuster si nécessaire, la concentration en sel à la valeur désirée.

La solution ainsi obtenue peut être utilisée directement dans une installation de polymérisation ou être stockée dans un réservoir de stockage 12 ou dans des conteneurs adaptés pour le transport.

En se référant à la figure 2, un second mode de réalisation du procédé de l'invention est décrit. Ce second mode de réalisation concerne un procédé fonctionnant selon un mode continu. Comme dans le premier mode de réalisation, le procédé comprend une première étape de dissolution de l'acide adipique mise en oeuvre dans le réacteur 14. L'acide adipique est alimenté par un système à vis sans fin 15, simultanément à de l'eau 16 et à de la diamine 30 sous forme pure ou solution aqueuse pour obtenir dans le réacteur 14 une solution contenant un rapport molaire diacide/diamine compris entre 1,5 et 5, de préférence voisin de 2,4, et une concentration pondérale en espèces dissoutes comprise entre 40 et 75%, par exemple égale à 57%.

Pour obtenir une homogénéisation de la solution dans le réacteur 14, une boucle de circulation externe 18 comprenant une pompe 19 est illustrée. Le réacteur 14 est également muni d'une agitation mécanique (non représentée). Une partie de la solution circulant dans la boucle alimente un réacteur 20.

Comme dans le premier mode de réalisation, le procédé comprend une étape de dissolution de l'acide téréphtalique mise en oeuvre dans le réacteur 21. L'acide téréphtalique est alimenté par un système à vis sans fin 29, simultanément à de l'eau 16 et à de la diamine 26 sous forme pure ou solution aqueuse pour obtenir dans le réacteur 21 une solution contenant un rapport molaire diacide/diamine inférieur à 1, par exemple voisin de 0,48, et une concentration pondérale en espèces dissoutes par exemple égale à 49%.

Pour obtenir une homogénéisation de la solution dans le réacteur 21, une boucle de circulation externe 17 comprenant une pompe 27 est illustrée. Le réacteur 21 est également muni d'une agitation mécanique (non représentée). Une partie 22 de la solution circulant dans la boucle alimente un réacteur 20.

Le réacteur 20 est équipé d'une boucle externe 28 de neutralisation comprenant une pompe 25.

Comme dans le premier mode de réalisation, la chaleur dégagée par la neutralisation permet une augmentation de la température de la solution jusqu'à atteindre, au maximum, la température d'ébullition de la solution à la pression opératoire.

Pour condenser l'eau ainsi évaporée, un condenseur 23 est prévu sur le réacteur 20.

La solution produite dans le réacteur 20 est dirigée (flux 24) vers des stockeurs non représentés.

Les exemples ci-dessous illustrent plus clairement le procédé de l'invention et les caractéristiques et avantages de celui-ci.

### EXEMPLES

### Exemple 1 : Production d'une solution aqueuse de Sel 66/6T 66/34 (proportion molaire) à 52% en poids selon le procédé discontinu.

### Préparation de la solution (A')

Une solution aqueuse d'acide téréphtalique et d'hexaméthylène diamine est préparée en ajoutant de l'acide téréphtalique (11,2 kg), à un liquide dont la température est égale à 80°C, ce dernier étant obtenu par ajout d'hexaméthylène diamine (18,2 kg d'une solution aqueuse à 90% en poids) et d'eau déminéralisée (27,4 kg) dans le réacteur 5 ; ce réacteur contient un pied de 22 kg d'une solution aqueuse d'acide téréphtalique, d'acide adipique (dans la proportion molaire 34/66) et d'hexaméthylène diamine présentant un rapport molaire diacides/diamine = 1,017 à une température de 103°C et une concentration pondérale en espèces dissoutes proche de 52%.

Avantageusement ce pied de solution est une faible partie de la solution (A) obtenue dans le réacteur 5 dans une opération de fabrication antérieure.

Le temps d'ajout de l'acide téréphtalique est de l'ordre de 4 minutes et sa salification avec l'hexaméthylène diamine conduit à une élévation de température du milieu. Le réacteur est muni d'une agitation mécanique. La dissolution de l'acide téréphtalique est obtenue au bout d'une minute après la fin d'introduction de l'acide téréphtalique. La solution aqueuse obtenue présente une concentration en espèces dissoutes de 49,5% en poids et la température finale de la solution est de 95°C.

### Préparation de la solution (B")

Une solution aqueuse d'acide adipique et d'hexaméthylène diamine est préparée en ajoutant simultanément de l'acide adipique en poudre (18,8 kg) et de l'hexaméthylène diamine (6,7 kg d'une solution aqueuse à 90% en poids à une température de 45°C) dans le réacteur 1, isolé thermiquement, contenant une solution aqueuse obtenue par addition d'eau (18 kg) dans un pied de 14 kg de solution aqueuse d'acide adipique et d'hexaméthylène diamine présentant un rapport molaire AA/HMD = 2,5 à une température de 63°C et une concentration pondérale en espèces dissoutes de 57%.

Avantageusement ce pied de solution est une faible partie de la solution (B") obtenue dans le réacteur 1 dans une opération de fabrication antérieure. Le réacteur est muni d'une agitation mécanique. En fin de préparation, les espèces dissoutes (57% en poids) consistent en 75,6% en poids d'acide adipique et 24,4% en poids d'hexaméthylène diamine. La température finale de la solution est de 63°C.

### Préparation de la solution (A)

43,6 kg (soit environ 75,7%) de la solution (B") préparée précédemment sont alors transférés dans le réacteur 5 de la figure 1, isolé thermiquement et équipé d'un condenseur 8. Le rapport diacides/diamine de la solution obtenue est proche de la stoechiométrie (rapport molaire diacides/HMD = 1,017).

L'énergie ou chaleur dégagée par la réaction de neutralisation provoque l'élévation de la température du milieu jusqu'à la température d'ébullition, à savoir 103°C environ dans l'exemple décrit. Les vapeurs produites sont condensées dans le condenseur 8 et forment un reflux total dans le réacteur 5. L'énergie évacuée par la condensation des vapeurs correspond à l'énergie de neutralisation excédentaire.

La concentration et le pH de la solution sont ensuite ajustés par ajout de 0,34 kg d'une solution aqueuse d'HMD à 90% en poids et à une température de 45°C, après transfert d'une partie de la solution (100 kg) dans un troisième réacteur 10. A la fin de cette étape, la solution est une solution aqueuse contenant 52% en poids de sel 66/6T avec un rapport molaire diacides/HMD égal à 1,003 et un pH égal à 7,20. Le pH est mesuré à 40°C sur un échantillon de la solution diluée avec de l'eau pour obtenir une concentration en espèces dissoutes égale à 100 g/L.

La solution obtenue est ensuite stockée dans un réservoir 12 illustré à la figure 1.

### Exemple 2: Production d'une solution aqueuse de Sel 66/6T 66/34 (proportion molaire) à 52% en poids selon le procédé discontinu.

### Préparation de la solution (A')

Une solution aqueuse d'acide téréphtalique et d'hexaméthylène diamine est préparée en ajoutant de l'acide téréphtalique (11,2 kg), à un liquide dont la température est égale à 80°C, ce dernier étant obtenu par ajout d'hexaméthylène diamine (24,9 kg d'une solution aqueuse à 90% en poids) et d'eau déminéralisée (27,4 kg) dans le réacteur 5, ce dernier contenant un pied de 22kg d'une solution aqueuse d'acide téréphtalique, d'acide adipique (dans la proportion molaire 34/66) et d'hexaméthylène diamine présentant un rapport molaire diacides/diamine = 1,017 à une température de 103°C et une concentration pondérale en espèces dissoutes proche de 52%.

Avantageusement ce pied de solution est une faible partie de la solution (A) obtenue dans le réacteur 5 dans une opération de fabrication antérieure.

Le temps d'ajout de l'acide téréphtalique est de l'ordre de 4 minutes et sa salification avec l'hexaméthylène diamine conduit à une élévation de température du milieu. Le réacteur est muni d'une agitation mécanique. La dissolution de l'acide téréphtalique est obtenue au bout de deux minutes après la fin d'introduction de l'acide téréphtalique. La solution aqueuse obtenue présente une concentration en espèces dissoutes de 52,7% en poids et la température finale de la solution est de 96°C.

### Préparation de la bouillie (B")

Une bouillie aqueuse d'acide adipique est préparée en ajoutant de l'acide adipique en poudre (18,8 kg) dans le réacteur 1, isolé thermiquement et maintenu à 70°C par chauffage, le réacteur contenant une bouillie aqueuse obtenue par ajout d'eau (18 kg) dans un pied de 14 kg de bouillie aqueuse d'acide adipique à une température de 70°C et une concentration pondérale en acide adipique (solide+dissous) de 51 %.

Avantageusement ce pied de bouillie est une faible partie de la bouillie (B") obtenue dans le réacteur 1 dans une opération de fabrication antérieure. Le réacteur est muni d'une agitation mécanique. En fin de préparation, la bouillie aqueuse présente une concentration en acide adipique (solide+dissous) de 51 % en poids. La température finale de la bouillie est de 70°C.

### Préparation de la solution (A)

36,8 kg (soit environ 72,4%) de la bouillie (B") préparée précédemment sont alors transférés dans le réacteur 5 de la figure 1, isolé thermiquement et équipé d'un condenseur 8. Le rapport diacides/diamine de la solution obtenue est proche de la stoechiométrie (rapport molaire diacides/HMD = 1,017).

L'énergie ou chaleur dégagée par la réaction de neutralisation provoque l'élévation de la température du milieu jusqu'à la température d'ébullition, à savoir 103°C environ dans l'exemple décrit. Les vapeurs produites sont condensées dans le condenseur 8 et forment un reflux total dans le réacteur 5. L'énergie évacuée par la condensation des vapeurs correspond à l'énergie de neutralisation excédentaire.

La concentration et le pH de la solution sont ensuite ajustés par ajout de 0,34 kg d'une solution aqueuse d'HMD à 90% en poids et à une température de 45°C, après transfert d'une partie de la solution (100 kg) dans un troisième réacteur 10. A la fin de cette étape, la solution est une solution aqueuse contenant 52% en poids de sel 66/6T avec un rapport molaire diacides/HMD égal à 1,003 et un pH égal à 7,20. Le pH est mesuré à 40°C sur un échantillon de la solution diluée avec de l'eau pour obtenir une concentration en espèces dissoutes égale à 100 g/L.

La solution obtenue est ensuite stockée dans un réservoir 12 illustré à la figure 1.

### Exemple 3: Production d'une solution aqueuse de Sel 66/6T 56/44 (proportion molaire) à 52% en poids selon le procédé discontinu.

### Préparation de la solution (A')

Une solution aqueuse d'acide téréphtalique et d'hexaméthylène diamine est préparée en ajoutant de l'acide téréphtalique (14,3 kg), à un liquide dont la température est égale à 80°C, ce dernier étant obtenu par ajout d'hexaméthylène diamine (19,1 kg d'une solution aqueuse à 90% en poids) et d'eau déminéralisée (30,4 kg) dans le réacteur 5, ce dernier contenant un pied de 22kg d'une solution aqueuse d'acide téréphtalique, d'acide adipique (dans la proportion molaire 44/56) et d'hexaméthylène diamine présentant un rapport molaire diacides/diamine = 1,017 à une température de 103°C et une concentration pondérale en espèces dissoutes proche de 52%.

Avantageusement ce pied de solution est une faible partie de la solution (A) obtenue dans le réacteur 5 dans une opération de fabrication antérieure.

Le temps d'ajout de l'acide téréphtalique est de l'ordre de 4 minutes et sa salification avec l'hexaméthylène diamine conduit à une élévation de température du milieu. Le réacteur est muni d'une agitation mécanique. La dissolution de l'acide téréphtalique est obtenue au bout de trois minutes après la fin d'introduction de l'acide téréphtalique. La solution aqueuse obtenue présente une concentration en espèces dissoutes de 50% en poids et la température finale de la solution est de 95°C.

### Préparation de la solution (B")

Une solution aqueuse d'acide adipique et d'hexaméthylène diamine est préparée en ajoutant simultanément de l'acide adipique en poudre (15,9 kg) et de l'hexaméthylène diamine (5,7 kg d'une solution aqueuse à 90% en poids à une température de 45°C) dans le réacteur 1, isolé thermiquement, contenant une solution aqueuse obtenue par addition d'eau (15,1 kg) dans un pied de 14 kg de solution aqueuse d'acide adipique et d'hexaméthylène diamine présentant un rapport molaire AA/HMD = 2,5 à une température de 63°C et une concentration pondérale en espèces dissoutes de 57%.

Avantageusement ce pied de solution est une faible partie de la solution (B") obtenue dans le réacteur 1 dans une opération de fabrication antérieure. Le réacteur est muni d'une agitation mécanique. En fin de préparation, les espèces dissoutes (57% en poids) consistent en 75,6% en poids d'acide adipique et 24,4% en poids d'hexaméthylène diamine. La température finale de la solution est de 63°C.

### Préparation de la solution (A)

36,7 kg (soit environ 72,4%) de la solution (B") préparée précédemment sont alors transférés dans le réacteur 5 de la figure 1, isolé thermiquement et équipé d'un condenseur 8. Le rapport diacides/diamine de la solution obtenue est proche de la stoechiométrie (rapport molaire diacides/HMD = 1,017).

L'énergie ou chaleur dégagée par la réaction de neutralisation provoque l'élévation de la température du milieu jusqu'à la température d'ébullition, à savoir 103°C environ dans l'exemple décrit. Les vapeurs produites sont condensées dans le condenseur 8 et forment un reflux total dans le réacteur 5. L'énergie évacuée par la condensation des vapeurs correspond à l'énergie de neutralisation excédentaire.

La concentration et le pH de la solution sont ensuite ajustés par addition de 0,34 kg d'une solution aqueuse d'HMD à 90% en poids et à une température de 45°C, après transfert d'une partie de la solution (100 kg) dans un troisième réacteur 10. A la fin de cette étape, la solution est une solution aqueuse contenant 52% en poids de sel 66/6T avec un rapport molaire diacides/HMD égal à 1,003.

La solution obtenue est ensuite stockée dans un réservoir 12 illustré à la figure 1.

### Exemple 4: Production d'une solution aqueuse de Sel 66/6T 66/34 (proportion molaire) à 52% en poids selon le procédé discontinu.

### Préparation de la solution (A')

Une solution aqueuse d'acide téréphtalique et d'hexaméthylène diamine est préparée en ajoutant de l'acide téréphtalique (11,2 kg), à un liquide dont la température est égale à 80°C, ce dernier étant obtenu par ajout d'hexaméthylène diamine (18, 2 kg d'une solution aqueuse à 90% en poids) et d'eau déminéralisée (27,4 kg) dans le réacteur 5.

Le temps d'ajout de l'acide téréphtalique est de l'ordre de 4 minutes et sa salification avec l'hexaméthylène diamine conduit à une élévation de température du milieu. Le réacteur est muni d'une agitation mécanique. La dissolution de l'acide téréphtalique est obtenue au bout d'une minute après la fin d'introduction de l'acide téréphtalique. La solution aqueuse obtenue présente une concentration en espèces dissoutes de 48,5% en poids et la température finale de la solution est de 95°C.

### Exemple 5 (comparatif) : Production d'une solution aqueuse de Sel 66/6T 66/34 (proportion molaire) à 52% en poids selon le procédé discontinu.

### Préparation de la solution (A')

Une solution aqueuse d'acide téréphtalique et d'hexaméthylène diamine est préparée en ajoutant de l'acide téréphtalique (11,2 kg) ), à un liquide dont la température est égale à 50°C, ce dernier étant obtenu par ajout d'hexaméthylène diamine (18, 2 kg d'une solution aqueuse à 90% en poids) et d'eau déminéralisée (27,4 kg), dans le réacteur 5.

Le temps d'ajout de l'acide téréphtalique est de l'ordre de 4 minutes et sa salification avec l'hexaméthylène diamine conduit à une très légère élévation de température du milieu. Le réacteur est muni d'une agitation mécanique.

L'ajout de l'acide téréphtalique conduit à la formation d'agglomérats de taille importante dont la dissolution n'est pas obtenue au bout de plusieurs heures (en maintenant la température à 50°C).

Ce mode de préparation ne permet pas l'obtention d'une solution (A') homogène, par rapport au mode de préparation selon l'exemple 4 de l'invention par exemple.

Ce mode de préparation ne convient pas à la préparation d'une solution homogène.

## Revendications

1. Procédé de fabrication d'une solution aqueuse (A) de sels de diacides et diamine(s) obtenus par mélange d'au moins deux diacides et d'au moins une diamine à une concentration pondérale en sel comprise entre 40 et 70% **caractérisé en ce qu'**il comprend les étapes suivantes :
- produire dans un réacteur une solution aqueuse (A') de diamine(s) et diacide(s), ayant un rapport molaire diacide/diamine inférieur à 1, par alimentation dans ledit réacteur contenant un liquide à une température comprise entre 55 et 95°C (bornes incluses), comprenant de l'eau et de la diamine, d'un flux (B') comprenant un diacide, éventuellement d'un flux comprenant de la diamine, éventuellement d'un flux comprenant de l'eau ; les débits du ou des flux d'alimentation étant contrôlé(s) pour avoir constamment une température de la solution dans le réacteur inférieure à la température d'ébullition sous la pression opératoire de celle-ci ; les quantités d'eau et de diamine du liquide ainsi que les débits des flux d'alimentation étant contrôlés pour avoir constamment un rapport molaire diacide(s)/diamine(s) inférieur à 1 ; le diacide du flux (B') étant un diacide aliphatique ou cycloaliphatique comprenant un nombre d'atomes de carbone supérieur à 10 ou un diacide aromatique ;
- mélanger la solution aqueuse (A') issue de la première étape avec un flux (B") comprenant au moins un diacide, le diacide étant un diacide aliphatique ou cycloaliphatique comprenant un nombre d'atomes de carbone inférieur ou égal à 10, et éventuellement de l'eau et/ou de la diamine complémentaires ; afin d'obtenir une solution aqueuse issue du mélange de (A') et (B") ayant un rapport molaire diacides/diamine(s) compris entre 0,9 et 1,1 ; cette solution étant portée à une température au plus égale à la température d'ébullition de la solution à la pression opératoire par au moins le dégagement de chaleur de la réaction entre la(les) diamine(s) et les diacides ; ceci afin d'obtenir la solution (A) de diacides et diamine(s) à la concentration et à la composition désirées.

2. Procédé selon la revendication 1, dans lequel la solution aqueuse (A') de diamine(s) et diacide(s), a un rapport molaire diacide/diamine inférieur ou égal à 0,9.

3. Procédé selon la revendication 1 ou 2, **caractérisé en ce que** l'étape qui consiste à produire dans un réacteur une solution aqueuse (A') de diamine(s) et diacide(s), ayant un rapport molaire diacide/diamine inférieur à 1, se fait par alimentation dans ledit réacteur contenant un liquide à une température comprise entre 60 et 90°C (bornes incluses), comprenant de l'eau et de la diamine, d'un flux (B') comprenant un diacide, éventuellement d'un flux comprenant de la diamine, éventuellement d'un flux comprenant de l'eau ; les débits du ou des flux d'alimentation étant contrôlé(s) pour avoir constamment une température de la solution dans le réacteur inférieure à la température d'ébullition sous la pression opératoire de celle-ci ; les quantités d'eau et de diamine du liquide ainsi que les débits des flux d'alimentation étant contrôlés pour avoir constamment un rapport molaire diacide(s)/diamine(s) inférieur à 1 ; le diacide du flux (B') étant un diacide aliphatique ou cycloaliphatique comprenant un nombre d'atomes de carbone supérieur à 10 ou un diacide aromatique.

4. Procédé selon l'une des revendications précédentes, **caractérisé en ce que** la solution aqueuse issue du mélange de (A') et (B") a un rapport molaire diacides/diamine(s) compris entre 0,98 et 1,02.

5. Procédé selon l'une des revendications précédentes, **caractérisé en ce que** le réacteur est maintenu sous atmosphère exempte d'oxygène.

6. Procédé selon l'une des revendications précédentes, **caractérisé en ce que** la diamine de la solution (A') et du flux (B") est l'hexaméthylène diamine.

7. Procédé selon l'une des revendications précédentes, **caractérisé en ce que** le diacide du flux (B') est l'acide téréphtalique.

8. Procédé selon l'une des revendications précédentes, **caractérisé en ce que** le diacide du flux (B") est l'acide adipique.

9. Procédé selon l'une des revendications précédentes, **caractérisé en ce que** la solution (A) présente une proportion molaire d'acide téréphtalique par rapport aux diacides comprise entre 5 et 80%.

10. Procédé selon l'une des revendications précédentes, **caractérisé en ce que** la solution (A) présente une proportion molaire d'acide téréphtalique par rapport aux diacides comprise entre 20 et 50%.

11. Procédé selon l'une des revendications précédentes, **caractérisé en ce que** le liquide comprend la totalité de l'eau et de la diamine de la solution (A').

12. Procédé selon l'une des revendications précédentes, **caractérisé en ce que** le flux (B") est une solution aqueuse de diacide et de diamine avec un rapport molaire diacide/diamine compris entre 1,5 et 5 et une concentration dans l'eau des espèces dissoutes comprise entre 40 et 75%.

13. Procédé selon l'une des revendications précédentes, **caractérisé en ce que** le flux (B") est une solution aqueuse de diacide et de diamine avec un rapport molaire diacide/diamine compris entre 1,5 et 5 et une concentration dans l'eau des espèces dissoutes comprise entre 45 et 65%.

## Patentansprüche

1. Verfahren zur Herstellung einer wässrigen Lösung (A) von Salzen von Disäuren und Diamin(en), erhalten durch Mischen von mindestens zwei Disäuren und mindestens einem Diamin in einer Salzkonzentration zwischen 40 und 70 Gew.-%, **dadurch gekennzeichnet, dass** es die folgenden Schritte umfasst:
- Herstellen einer wässrigen Lösung (A') von Diamin(en) und Disäure(n) mit einem Disäure/Diamin-Molverhältnis von weniger als 1 in einem Reaktor durch Zufuhr eines Stroms (B'), der eine Disäure umfasst, gegebenenfalls eines Stroms, der Diamin umfasst, und gegebenenfalls eines Stroms, der Wasser umfasst, zu dem eine Flüssigkeit enthaltenden Reaktor bei einer Temperatur zwischen 55 und 95°C (Grenzen eingeschlossen); wobei die Strömungsraten des Zufuhrstroms bzw. der Zufuhrströme so gesteuert werden, dass ständig eine Temperatur der Lösung im Reaktor vorliegt, die unter der Siedetemperatur der Lösung unter dem Betriebsdruck liegt; wobei die Mengen von Wasser und Diamin in der Flüssigkeit sowie die Strömungsraten der Zufuhrströme so gesteuert werden, dass ständig ein Disäure(n)/Diamin(e)-Molverhältnis von weniger als 1 vorliegt; wobei es sich bei der Disäure des Stroms (B') um eine aliphatische oder cycloaliphatische Disäure mit mehr als 10 Kohlenstoffatomen oder eine aromatische Disäure handelt;
- Mischen der wässrigen Lösung (A') aus dem ersten Schritt mit einem Strom (B''), der mindestens eine Disäure, wobei es sich bei der Disäure um eine aliphatische oder cycloaliphatische Disäure mit kleiner gleich 10 Kohlenstoffatomen handelt, und gegebenenfalls Wasser und/oder zusätzliches Diamin umfasst; wobei man eine wässrige Lösung, die sich aus dem Mischen von (A') und (B'') ergibt, mit einem Disäuren/Diamin(e)-Molverhältnis zwischen 0,9 und 1,1 erhält; wobei diese Lösung durch zumindest die Freisetzung der Wärme der Reaktion zwischen dem Diamin bzw. den Diaminen und den Disäuren auf eine Temperatur, die höchstens gleich der Siedetemperatur der Lösung unter dem Betriebsdruck ist, gebracht wird; wodurch man die Lösung (A) von Disäuren und Diamine(n) mit der gewünschten Konzentration und der gewünschten Zusammensetzung erhält.

2. Verfahren nach Anspruch 1, bei dem die wässrige Lösung (A') von Diamin(en) und Disäure(n) ein Disäure/Diamin-Molverhältnis kleiner gleich 0,9 aufweist.

3. Verfahren nach Anspruch 1 oder 2, **dadurch gekennzeichnet, dass** der Schritt, der aus dem Herstellen einer wässrigen Lösung (A') von Diamin(en) und Disäure(n) mit einem Disäure/Diamin-Molverhältnis von weniger als 1 in einem Reaktor besteht, durch Zufuhr eines Stroms (B'), der eine Disäure umfasst, gegebenenfalls eines Stroms, der Diamin umfasst, und gegebenenfalls eines Stroms, der Wasser umfasst, zu dem eine Flüssigkeit enthaltenden Reaktor bei einer Temperatur zwischen 60 und 90°C (Grenzen eingeschlossen) erfolgt; wobei die Strömungsraten des Zufuhrstroms bzw. der Zufuhrströme so gesteuert werden, dass ständig eine Temperatur der Lösung im Reaktor vorliegt, die unter der Siedetemperatur der Lösung unter dem Betriebsdruck liegt; wobei die Mengen von Wasser und Diamin in der Flüssigkeit sowie die Strömungsraten der Zufuhrströme so gesteuert werden, dass ständig ein Disäure(n)/Diamin(e)-Molverhältnis von weniger als 1 vorliegt; wobei es sich bei der Disäure des Stroms (B') um eine aliphatische oder cycloaliphatische Disäure mit mehr als 10 Kohlenstoffatomen oder eine aromatische Disäure handelt.

4. Verfahren nach einem der vorhergehenden Ansprüche, **dadurch gekennzeichnet, dass** die wässrige Lösung, die sich aus dem Mischen von (A') und (B") ergibt, ein Disäuren/Diamin(e)-Molverhältnis zwischen zwischen 0,98 und 1,02 aufweist.

5. Verfahren nach einem der vorhergehenden Ansprüche, **dadurch gekennzeichnet, dass** der Reaktor unter einer sauerstofffreien Atmosphäre gehalten wird.

6. Verfahren nach einem der vorhergehenden Ansprüche, **dadurch gekennzeichnet, dass** es sich bei dem Diamin der Lösung (A') und des Stroms (B") um Hexamethylendiamin handelt.

7. Verfahren nach einem der vorhergehenden Ansprüche, **dadurch gekennzeichnet, dass** es sich bei der Disäure des Stroms (B') um Terephthalsäure handelt.

8. Verfahren nach einem der vorhergehenden Ansprüche, **dadurch gekennzeichnet, dass** es sich bei der Disäure des Stroms (B") um Adipinsäure handelt.

9. Verfahren nach einem der vorhergehenden Ansprüche, **dadurch gekennzeichnet, dass** die Lösung (A) einen Molanteil an Terephthalsäure, bezogen auf die Disäuren, zwischen 5 und 80% aufweist.

10. Verfahren nach einem der vorhergehenden Ansprüche, **dadurch gekennzeichnet, dass** die Lösung (A) einen Molanteil an Terephthalsäure, bezogen auf die Disäuren, zwischen 20 und 50% aufweist.

11. Verfahren nach einem der vorhergehenden Ansprüche, **dadurch gekennzeichnet, dass** die Flüssigkeit das gesamte Wasser und Diamin der Lösung (A') umfasst.

12. Verfahren nach einem der vorhergehenden Ansprüche, **dadurch gekennzeichnet, dass** es sich bei dem Strom (B") um eine wässrige Lösung von Disäure und Diamin mit einem Disäure/Diamin-Molverhältnis zwischen 1,5 und 5 und einer Konzentration gelöster Spezies in dem Wasser zwischen 40 und 75% handelt.

13. Verfahren nach einem der vorhergehenden Ansprüche, **dadurch gekennzeichnet, dass** es sich bei dem Strom (B") um eine wässrige Lösung von Disäure und Diamin mit einem Disäure/Diamin-Molverhältnis zwischen 1,5 und 5 und einer Konzentration gelöster Spezies in dem Wasser zwischen 45 und 65% handelt.

## Claims

1. Process for the manufacture of an aqueous solution (A) of salts of diacids and diamine(s) which are obtained by mixing at least two diacids and at least one diamine at a concentration by weight of salt of between 40 and 70%, **characterized in that** it comprises the following stages :
- producing, in a reactor, an aqueous solution (A') of diamine(s) and diacid(s) having a diacid/diamine molar ratio of less than 1 by feeding, to the said reactor comprising a liquid at a temperature of between 55 and 95°C (limits included), comprising water and diamine, a stream (B') comprising a diacid, optionally a stream comprising diamine and optionally a stream comprising water; the flow rates of the feed stream or streams being controlled in order to always have a temperature of the solution in the reactor which is less than the boiling point of the solution under the operating pressure; the amounts of water and of diamine in the liquid and the flow rates of the feed streams being controlled in order to always have a diacid(s)/diamine(s) molar ratio of less than 1 ; the diacid of the stream (B') being an aliphatic or cycloaliphatic diacid comprising a number of carbon atoms of greater than 10 or an aromatic diacid;
- mixing the aqueous solution (A') resulting from the first stage with a stream (B") comprising at least one diacid, the diacid being an aliphatic or cycloaliphatic diacid comprising a number of carbon atoms of less than or equal to 10, and optionally supplementary water and/or supplementary diamine; in order to obtain an aqueous solution (resulting from the mixing of (A') and (B")) having a diacids/diamine(s) molar ratio of between 0.9 and 1.1; this solution being brought to a temperature at most equal to the boiling point of the solution at the operating pressure by at least the release of heat of the reaction between the diamine(s) and the diacids; in order to obtain the solution (A) of diacids and diamine(s) at the desired concentration and the desired composition.

2. Process according to Claim 1, in which the aqueous solution (A') of diamine(s) and diacid(s) has a diacid/diamine molar ratio of less than or equal to 0.9.

3. Process according to Claim 1 or 2, **characterized in that** the stage which consists in producing, in a reactor, an aqueous solution (A') of diamine(s) and diacid(s) having a diacid/diamine molar ratio of less than 1 is carried out by feeding, to the said reactor comprising a liquid at a temperature of between 60 and 90°C (limits included), comprising water and diamine, a stream (B') comprising a diacid, optionally a stream comprising diamine and optionally a stream comprising water; the flow rates of the feed stream or streams being controlled in order to always have a temperature of the solution in the reactor which is less than the boiling point of the solution under the operating pressure; the amounts of water and of diamine in the liquid and the flow rates of the feed streams being controlled in order to always have a diacid(s)/diamine(s) molar ratio of less than 1 ; the diacid of the stream (B') being an aliphatic or cycloaliphatic diacid comprising a number of carbon atoms of greater than 10 or an aromatic diacid.

4. Process according to one of the preceding claims, **characterized in that** the aqueous solution resulting from the mixing of (A') and (B") has a diacids/diamine(s) molar ratio of between 0.98 and 1.02.

5. Process according to one of the preceding claims, **characterized in that** the reactor is maintained under an atmosphere devoid of oxygen.

6. Process according to one of the preceding claims, **characterized in that** the diamine of the solution (A') and of the stream (B") is hexamethylenediamine.

7. Process according to one of the preceding claims, **characterized in that** the diacid of the stream (B") is terephthalic acid.

8. Process according to one of the preceding claims, **characterized in that** diacid of the stream (B") is adipic acid.

9. Process according to one of the preceding claims, **characterized in that** the solution (A) exhibits a molar proportion of terephthalic acid, with respect to the diacids, of between 5 and 80%. '

10. Process according to one of the preceding claims, **characterized in that** the solution (A) exhibits a molar proportion of terephthalic acid, with respect to the diacids, of between 20 and 50%.

11. Process according to one of the preceding claims, **characterized in that** the liquid comprises all the water and diamine of the solution (A').

12. Process according to one of the preceding claims, **characterized in that** the stream (B") is an aqueous solution of diacid and of diamine with a diacid/diamine molar ratio of between 1.5 and 5 and a concentration in the water of the dissolved entities of between 40 and 75%.

13. Process according to one of the preceding claims, **characterized in that** the stream (B") is an aqueous solution of diacid and of diamine with a diacid/diamine molar ratio of between 1.5 and 5 and a concentration in the water of the dissolved entities of between 45 and 65%.
